# EUROPEAN PATENT SPECIFICATION

(11) **EP 1 584 337 B1**
(45) Date of publication and mention of the grant of the patent: **02.11.2016**
(21) Application number: 04700515.2
(22) Date of filing: 07.01.2004
(51) Int. Cl.: A61L 27/06, A61L 27/56

(54) **ARTIFICIAL BONE CAPABLE OF INDUCING NATURAL BONE AND METHOD FOR PREPARATION THEREOF**
SYNTHETISCHER KNOCHEN, DER NATÜRLICHEN KNOCHEN INDUZIEREN KANN, UND HERSTELLUNGSVERFAHREN DAFÜR
OS ARTIFICIEL CAPABLE D'INDUIRE UN OS NATUREL ET PROCÉDÉ D'ÉLABORATION

(30) Priority: 10.01.2003 JP 2003004028
(43) Date of publication of application: 12.10.2005
(73) Proprietor: Osaka Yakin Kogyo Co., Ltd., Osaka city Osaka 533-0005 (JP)
(72) Inventor: NAKAMURA, Takashi, 6150005 (JP); KOKUBO, Tadashi, 6170841 (JP); MATSUSHITA Tomiharu,, 6638107 (JP); FUJIBAYASHI, Shunsuke, 5650862 (JP); KIM, Hyun-Min, Pundang-Ku, Seongnam-Shi, Kyunggi-Do (KR)
(74) Representative: Hart-Davis, Jason
(86) International application number: PCT/JP2004/000042
(87) International publication number: WO 2004/062705

(56) References cited:
- WO-A1-98/51231
- JP-A- 5 056 990
- JP-A- 5 131 024
- JP-A- 2000 210 313
- JP-A- 2000 210 313
- JP-A- 2003 190 272
- US-A- 5 609 633
- KIM H.M. ET AL: 'Bioactive macroporous titanium surface layer on titanium substrate' JOURNAL OF BIOMEDICAL MATERIALS RESEARCH vol. 52, no. 3, December 2000, pages 553 - 557, XP002979951

## Description

### TECHNICAL FIELD

The present invention relates to an artificial bone comprising a lump of titanium or titanium alloy, and particularly an osteoinductive artificial bone and a manufacturing method thereof.

### BACKGROUND ART

[Patent Document 1] Japanese Patent No.2775523
[Patent Document 2] JP2002-102330,A
[Non Patent Literature Document 1] J. Biomed. Mater. Res. (Appl. Biomater.), 58, 270-276 (2001)

Titanium or titanium alloy (hereinafter referred to simply as titanium or the like) has been utilized as a material for an artificial bone by reason of having less toxicity to a living body. The following have conventionally been known as materials for an artificial bone comprising titanium or the like: a material such that a film comprising amorphous alkali titanate is formed on a surface of titanium or the like, on which film a second film comprising apatite is formed as required (Patent Document 1) ; and a material such that a film comprising anatase is formed on a surface of titanium or the like, on which film a second film comprising apatite is formed as required (Patent Document 2). These materials for an artificial bone are both superior in bonding ability with a living bone. That is to say, when these materials for an artificial bone are implanted into defect of living bone a surface of the materials for an artificial bone bonds firmly to neighboring living bone contacting therewith.

On the other hand, it has been known that a specific ceramic porous body comprising hydroxyl apatite etc. has osteoinductivity, which can induce bone formation even in a location in which a bone does not intrinsically exist, for example, in muscle (Non Patent Literature Document 1).

According to WO 98/51231, an implant element for permanent anchorage in bone tissue is made of titanium with a titanium oxide surface which has been modified by anodization

According to US patent 5,609,633, a bone substitute, wherein titanium or titanium alloys and apatite are tightly adhered, is produced by soaking a substrate of titanium or titanium alloy in an alkaline solution to form a layer comprising amorphous alkali titanate, followed by the heating of the said substrate up to at most the titanium or titanium alloy transition temperature so as to form a stabilized amorphous alkali titanate layer.

According to JP 2000-210313 A, a bone substitutive material is formed with ruggedness on the surface of titanium or titanium alloy which is a bonding surface to the vital tissue.

JP-H05-131024 A shows an organism compatible material comprising a porous body of a TiAl intermetallic compound with a pore and porosity of 5.0 % or less.

According to JP-H05-56990 A, coarse particles consisting of sponge titanium are stuck to the surface of implant base material made of a titanium alloy, partly or completely melted by plasma, and are utilized as an adhesive for sticking the fine particles at this time, and rugged films formed on the surface of the base material promote infiltration and sticking of biotissue.

H. Kim et al. in "Journal of Biomedical Materials Research, Volume 52, Issue 3, pages 553 - 557, 5 December 2000, show a bioactive macroporous titanium surface layer on titanium substrate to induce bonelike apatite formation.

According to JP 2003-190272 A, after a base material, at least the surface of which is formed of titanium or titanium alloy is washed and dried, anodic oxidation is performed taking a sample as an anode in an acidic solution, or heat treatment is performed subsequently to the anodic oxidation, thereby forming anodic oxidation layer having organism activity on the surface of the base material so as to manufacture bone substitute material.

### DISCLOSURE OF INVENTION

### [Problem to be solved by the Invention]

A ceramic porous body, however, is a fragile material having a compressive strength of approximately 10 to 30 MPa and a fracture toughness of 5 MPa·m^{1/2} or less, and thereby is implanted into living body and loaded, leading to failure. Accordingly, a region of application thereof is actually limited to an unloaded region.

### [Means for solving the problem]

An object of the present invention, therefore, is to provide a load-resistant and osteoinductive artificial bone.

In order to attain the object, an artificial bone of the present invention of claim 1 is provided with:
a porous body consisting of a lump of titanium or titanium alloy with a porosity of 30 to 80%, which has a film which is formed from at least one phase selected from an amorphous titanium oxide phase, an amorphous alkali titanate phase, an anatase phase and a rutile phase aligned with (101) plane. The porous body has pores communicating in a three-dimensional mesh network having diameters of 100 to 3000 µm, preferably 200 to 500 µm, and holes having a diameter of 50 µm or less on an inner surface of the pore.

The film is formed on a surface of the above-mentioned pores and holes in the porous body.

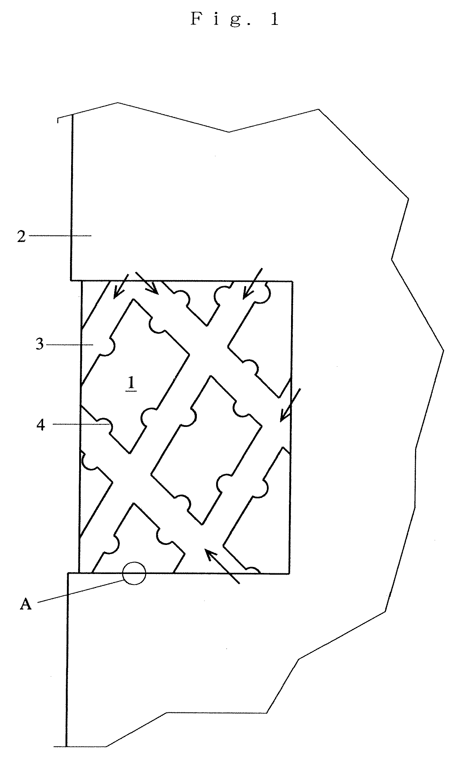

As schematically shown in Fig. 1, when an artificial bone 1 of the present invention is implanted into a living tissue 2, body fluid and cell (hereinafter referred to as "body fluid etc.") pass through a pore 3 as an arrow to permeate into an artificial bone 1. Body fluid etc. are captured by a hole 4 while passing through the pore 3. Any of the above-mentioned amorphous titanium oxide phase, amorphous alkali titanate phase, anatase phase and rutile phase aligned with (101) plane has apatite-forming ability in a living body. Accordingly, the body fluid etc. captured by the hole 4 react with a film (not shown in Fig.) formed in the hole 4 or in the periphery of the hole 4 to form a bone on the film. The artificial bone 1 remarkably differs in this respect from a conventional artificial bone comprising titanium or the like, which forms a bone only in a contacting portion with an living bone to bond therewith. That is to say, a conventional artificial bone has formed a new bone only in a contacting portion (for example, a portion A) between the artificial bone and a living tissue 2 in the case where the above-mentioned living tissue 2 is a living bone and a location for implanted is a bone defect. On the contrary, the artificial bone 1 of the present invention forms a new bone in a location away from a living tissue 2, such as in the hole 4 or in the periphery thereof.

The diameter of the pore 3 is even from a surface of the artificial bone 1 through the inside for the reason that Fig. 1 is a schematic view, which diameter is not necessarily even and is preferred to be within a range of 100 to 3000 µm, or rather actually not even. The diameter of the hole 4 is also diverse in the above-mentioned range.

A pore size less than 100 µm, however, causes body fluid etc. to pass through with difficulty, while a pore size more than 3000 µm causes too long years and months to be required for filling in the pore by a newly formed bone, and the diameter is thereby limited to a range of 100 to 3000 µm. A hole size exceeding 50 µm causes body fluid etc. to be captured with difficulty, and the diameter is thereby limited to 50 µm or less.

Among phases composing the above-mentioned film, anatase phase is the highest in the apatite-forming ability. Amorphous alkali titanate phase, meanwhile, is superior in long-term bond strength between apatite and titanium. In any case, the film preferably has a thickness of 0.1 to 10.0 µm. The reason therefor is that a thickness less than 0.1 µm brings a poor capability of forming a bone, while a thickness of 10.0 µm brings a sufficient capability of forming a bone.

An appropriate method of manufacturing an artificial bone of the present invention:
is characterized by immersing the above-mentioned porous body in an alkaline aqueous solution.

When a porous body comprising titanium or the like is immersed in an alkaline aqueous solution, the aqueous solution permeates into pores to form a film consisting essentially of amorphous alkali titanate on surfaces of the pores and holes. The porous body is thereafter immersed in water for changing this film to amorphous titanium oxide phase or anatase phase. Then, an alkali component of the titanate is exchanged for hydronium ions in water so as to be amorphous phase of titanium oxide or anatase phase. Warm water of 150 °C or less, preferably 30 to 90 °C, is used as this water. The time for immersing in warm water is rendered longer as water temperature is lower.

The above-mentioned porous body can be obtained by plasma-spraying titanium powder on a sprayed body. In this case, it is preferable that the titanium powder comprises a group of irregular particles and each of the particles is porous. The reason therefore is that a particle void and a pore in a particle can be controlled so as to be the above-mentioned pore and the above-mentioned hole, respectively. In addition, the above-mentioned titanium powder preferably comprises a fine powder having a particle diameter of 20 to 30 µm and a coarse powder having a particle diameter of 100 to 300 µm. The reason therefor is that the ratio therebetween allows a porous body having a desirable porosity to be obtained and a bond between particles to be strengthened.

When the above-mentioned porous body is heated after being immersed in the above-mentioned alkaline aqueous solution or after further subsequently being immersed in water, the fraction of amorphous alkali titanate phase or anatase phase is increased. This heating temperature is preferably 200 to 800 °C. The reason therefore is that a heating temperature less than 200 °C causes the crystallization into anatase phase to be brought with difficulty, while a heating temperature more than 800 °C causes mechanical strength to be lowered by reason of the phase change of titanium or the like and the progress of softening thereof.

Another appropriate method of manufacturing an artificial bone of the present invention:
is characterized by immersing the above-mentioned porous body in an electrolytic solution, preferably at voltage for causing spark discharge. In this case, the electrolytic solution is preferably an aqueous solution containing sulfuric acid or sulfate. The reason therefor is that the anodization in such an electrolytic solution allows the formation of a film with the coexistence of anatase phase and rutile phase aligned with (101) plane, which film with the coexistence of those two phases is particularly superior in the capability of forming apatite. In this specification, with regard to rutile, the case where peak intensity derived from (101) plane exceeds 1/2 of peak intensity derived from (110) plane is referred to as the alignment with (101) plane.

### [Effect of the Invention]

As described above, an artificial bone of the present invention can be a material for reinforcement or substitution in every location of a living body by reason of having high strength and osteoinductivity.

### BRIEF DESCRIPTION OF DRAWINGS

Fig. 1 is a view schematically showing a state of implanting an artificial bone of the present invention in a living body.
Fig. 2 is a view showing the pore-diameter distribution of a porous body applied to an artificial bone of an example.
Fig. 3 is a view showing the porosity of the above-mentioned porous body.
Fig. 4 is an approximately 120-times microphotograph of a stained section of the above-mentioned artificial bone implanted into a living body for 12 months.

### BEST MODE FOR CARRYING OUT THE INVENTION

### -Preliminary Experiment Example-

A titanium plate of 15 x 10 x 1 mm³ was immersed in a 5M-sodium hydroxide aqueous solution at a temperature of 60 °C for 24 hours, subsequently immersed in distilled water at a temperature of 40 °C for 48 hours, and thereafter heated at a temperature of 600 °C for 1 hour. When a surface of the obtained substrate was examined by thin-film X-ray diffraction, a film comprising anatase precipitated in large quantities was formed.

### -Example-

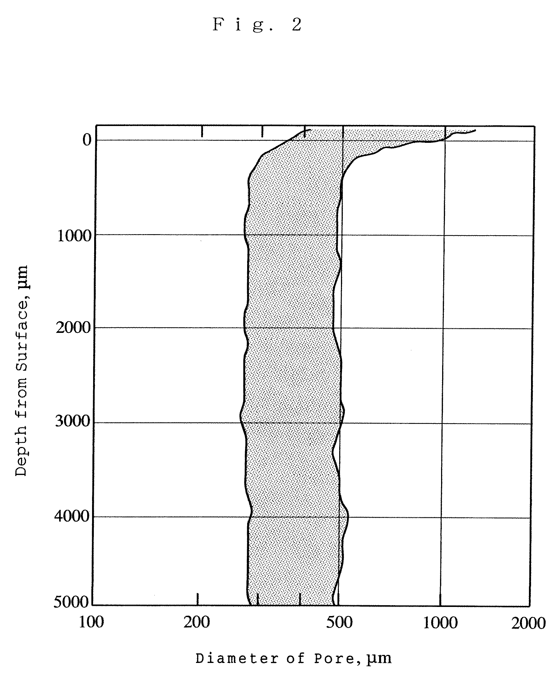

Mixed powder, in which an irregular titanium fine powder having a particle diameter of 20 to 30 µm and an irregular titanium coarse powder having a particle diameter of 100 to 300 µm were mixed at a ratio of 1 to 3, were prepared. A porous body having a thickness of approximately 10 mm was formed on a titanium plate by plasma-spraying the mixed powder thereon. A part of this porous body was cut out and ground. The diameter of pores existing on a ground surface was measured at every grinding of 0.1 mm (100 µm).
The results of measuring are shown in Fig. 2.

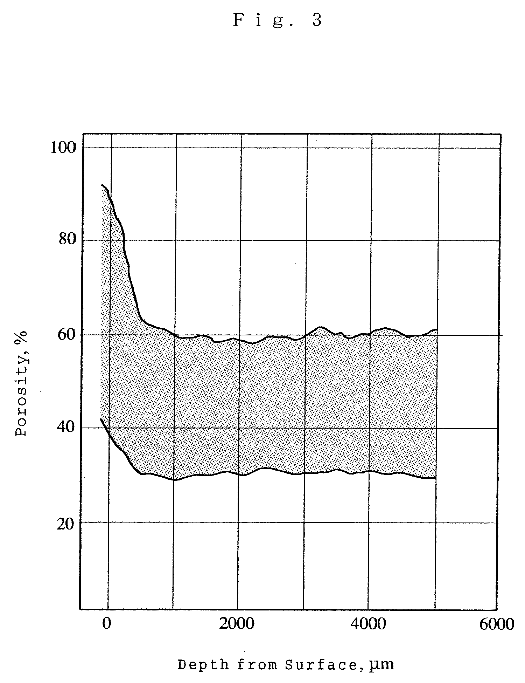

As shown in Fig. 2, the porous body had a multitude of communicating pores having a diameter of 300 to 500 µm in a range from a surface to a depth of 5 mm. The value obtained by dividing the total of areas of these pores by the total area of an observed surface was regarded as the porosity, which is shown in Fig. 3. As shown in Fig. 3, the porosity of the porous body was 30 to 60% in a range from a surface to a depth of 5 mm. When the ground surface was observed under a magnification of 100 times, the pores interconnected in a network and additionally holes having a diameter of approximately 0.1 to 10 µm existed in each particle.

Next, the residue of the above-mentioned porous body before being ground was cut out to a size of 5×5×7 mm, which was immersed in a 5M-sodium hydroxide aqueous solution at a temperature of 60 °C for 24 hours, subsequently immersed in distilled water at a temperature of 40 °C for 48 hours, and thereafter heated at a temperature of 600 °C for 1 hour.

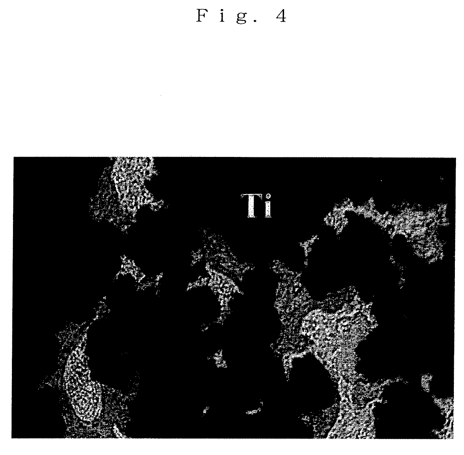

The obtained porous body was implanted into back muscle of a mature beagle and taken out after 12 months. When this was stained in toluidine blue and observed with an optical microscope, a new lamellar bone was found on an inner surface of pores in the porous body as shown in Fig. 4 as an approximately 120-times macrophotograph. In Fig. 4, the black portion (including the outline character portion of 'Ti' shape denoting titanium) is titanium, the dark gray portion is a newly formed bone and the light gray portion is an air void or soft tissue. The scanning electron microscope observation and energy-dispersive X-ray spectrum revealed that a newly formed bone bonded directly to a surface of titanium, and contained calcium and phosphorus. The pathological calcification was not found.

### -Comparative Example 1-

The same treatment as in the above-mentioned Example was performed except that a porous body obtained by plasma-spraying and cut out to a size of 5×5×7 mm was directly implanted into the back muscle of a beagle. As a result, the formation of a new-born bone was not found.

### -Comparative Example 2-

The same treatment as in the above-mentioned Example was performed except for replacing the porous body with a circular cylinder comprising a fibrous lump of titanium having an outer diameter of 4 mm, a length of 11 mm, a porosity of 40 to 60% and a pore size of 50 to 450 µm. As a result, the formation of a new-born bone was not found.

### -Comparative Example 3-

The same treatment as in Comparative Example 2 was performed except that a circular cylinder of Comparative Example 2 was immersed neither in a sodium hydroxide aqueous solution nor in distilled water and directly implanted into back muscle of a beagle. As a result, the formation of a newly formed bone was not found.

## Claims

1. An osteoinductive artificial bone (1) consisting of:
- a porous body consisting of a lump of titanium or titanium alloy with a porosity of 30 to 80%, which has
-- pores (3) communicating in a three-dimensional mesh network with diameters of 100 to 3000 µm and
-- holes (4) with a diameter of 50 µm or less at an inner surface of the pores (3); and
- a film formed in the porous body on a surface of the pores (3) and holes (4) which is formed from at least one phase selected from an amorphous titanium oxide phase, an amorphous alkali titanate phase, an anatase phase and a rutile phase aligned with (101) plane.

2. The artificial bone (1) of claim 1, wherein the film has a thickness of 0.1 to 10.0 µm.

3. The artificial bone (1) of claim 1 or 2, wherein the diameter of the pore is 200 to 500 µm.

4. A method of manufacturing an osteoinductive artificial bone (1), **characterized by**
- in an alkaline aqueous solution immersing of a porous body consisting of a lump of titanium or titanium alloy with a porosity of 30 to 80% which has
-- pores (3) communicating in a three-dimensional mesh network with diameters of 100 to 3000 µm and
-- holes (4) with a diameter of 50 µm or less at an inner surface of the pores (3).

5. The method of claim 4, wherein the body is obtained by plasma-spraying titanium powder on a sprayed body.

6. The method of claim 5, wherein the titanium powder comprises a group of irregular particles and each of the particles is porous.

7. The method of claim 5 or 6, wherein the titanium powder comprises a fine powder having a particle diameter of 20 to 30 µm and a coarse powder having a particle diameter of 100 to 300 µm.

8. The method of any one of claims 4 to 7, further comprising heating the body after the immersion.

9. The method of claim 8, wherein the heating temperature is 200 to 800°C.

10. The method of claim 8 or 9, further comprising immersing the body in water after the immersion in an alkaline aqueous solution before the heat.

11. A method of manufacturing an osteoinductive artificial bone (1), **characterized by**
- in an electrolytic solution anodizing of a porous body consisting of a lump of titanium or titanium alloy with a porosity of 30 to 80% which has
-- pores (3) communicating in a three-dimensional mesh network with diameters of 100 to 3000 µm and
-- holes (4) with a diameter of 50 µm or less at an inner surface of the pores (3).

## Patentansprüche

1. Osteoinduktiver künstlicher Knochen (1), bestehend aus:
- einem porösen Körper, der aus einer Titanmasse oder Titanlegierung besteht, mit einer Porosität von 30 bis 80 %, der aufweist:
-- Poren (3), die über ein dreidimensionales Maschennetz mit Durchmessern von 100 bis 3.000 µm in Verbindung stehen, und
-- Löcher (4) mit einem Durchmesser von 50 µm oder weniger an einer Innenfläche der Poren (3), und
- einem Film, der in dem porösen Körper auf einer Oberfläche der Poren (3) und Löcher (4) ausgebildet ist, der aus wenigstens einer Phase gebildet ist, die aus einer amorphen Titanoxidphase, einer amorphen Alkalititanatphase, einer Anatasphase und einer Rutilphase ausgewählt ist, der an einer Ebene (101) ausgerichtet ist.

2. Künstlicher Knochen (1) nach Anspruch 1, wobei der Film eine Dicke von 0,1 bis 10,0 µm aufweist.

3. Künstlicher Knochen (1) nach Anspruch 1 oder 2, wobei der Durchmesser der Pore 200 bis 500 µm beträgt.

4. Verfahren zum Herstellen eines osteoinduktiven künstlichen Knochens (1), **gekennzeichnet durch**
- Eintauchen eines porösen Körpers, der aus einer Titanmasse oder Titanlegierung besteht, mit einer Porosität von 30 bis 80 % in einer wässrigen Alkalilösung, der aufweist:
-- Poren (3), die über ein dreidimensionales Maschennetz mit Durchmessern von 100 bis 3.000 µm in Verbindung stehen, und
-- Löcher (4) mit einem Durchmesser von 50 µm oder weniger an einer Innenfläche der Poren (3).

5. Verfahren nach Anspruch 4, wobei der Körper durch Plasmaspritzen eines Titanpulvers auf einen Spritzkörper erhalten ist.

6. Verfahren nach Anspruch 5, wobei das Titanpulver eine Gruppe unregelmäßiger Partikel umfasst und jeder der Partikel porös ist.

7. Verfahren nach Anspruch 5 oder 6, wobei das Titanpulver ein Feinpulver, das einen Partikeldurchmesser von 20 bis 30 µm aufweist, und ein Grobpulver, das einen Partikeldurchmesser von 100 bis 300 µm aufweist, umfasst.

8. Verfahren nach einem der Ansprüche 4 bis 7, ferner umfassend Erwärmen des Körpers nach dem Eintauchen.

9. Verfahren nach Anspruch 8, wobei die Erwärmungstemperatur 200 bis 800 °C beträgt.

10. Verfahren nach Anspruch 8 oder 9, ferner umfassend Eintauchen des Körpers in Wasser nach dem Eintauchen in eine Alkalilösung vor dem Erwärmen.

11. Verfahren zum Herstellen eines osteoinduktiven künstlichen Knochens (1), **gekennzeichnet durch**
- Anodisieren eines porösen Körpers, der aus einer Titanmasse oder Titanlegierung besteht, mit einer Porosität von 30 bis 80 % in einer Elektrolytlösung, der aufweist:
-- Poren (3), die über ein dreidimensionales Maschennetz mit Durchmessern von 100 bis 3.000 µm in Verbindung stehen, und
-- Löcher (4) mit einem Durchmesser von 50 µm oder weniger an einer Innenfläche der Poren (3).

## Revendications

1. Os artificiel ostéo-inducteur (1) constitué de :
- un corps poreux consistant en un gros morceau de titane ou d'alliage de titane avec une porosité de 30 à 80 %, lequel présente
-- des pores (3) communiquant dans un réseau de grille tridimensionnel avec des diamètres de 100 à 3 000 µm et
-- des trous (4) avec un diamètre de 50 µm ou inférieur sur une surface interne des pores (3) ; et
- un film formé dans le corps poreux sur une surface des pores (3) et trous (4) qui est formé à partir d'au moins une phase qui est choisie parmi une phase amorphe d'oxyde de titane, une phase amorphe de titanate d'alcali, une phase anatase et une phase rutile alignée avec le plan (101).

2. Os artificiel (1) selon la revendication 1, dans lequel le film présente une épaisseur de 0,1 à 10,0 µm.

3. Os artificiel (1) selon la revendication 1 ou 2, dans lequel le diamètre du pore est de 200 à 500 µm.

4. Procédé de fabrication d'un os artificiel ostéo-inducteur (1),
**caractérisé par**
- l'immersion dans une solution aqueuse alcaline d'un corps poreux constitué d'un gros morceau de titane ou d'alliage de titane avec une porosité de 30 à 80 % qui présente
-- des pores (3) communiquant dans un réseau de grille tridimensionnel avec des diamètres de 100 à 3 000 µm et
-- des trous (4) avec un diamètre de 50 µm ou inférieur sur une surface interne des pores (3).

5. Procédé selon la revendication 4, dans lequel le corps est obtenu par pulvérisation au plasma de poudre de titane sur un corps pulvérisé.

6. Procédé selon la revendication 5, dans lequel la poudre de titane comprend un groupe de particules irrégulières et chacune des particules est poreuse.

7. Procédé selon la revendication 5 ou 6, dans lequel la poudre de titane comprend une fine poudre présentant un diamètre de particule de 20 à 30 µm et une poudre grossière présentant un diamètre de particule de 100 à 300 µm.

8. Procédé selon l'une quelconque des revendications 4 à 7, comprenant de plus le chauffage du corps après immersion.

9. Procédé selon la revendication 8, dans lequel la température de chauffage est de 200 à 800°C.

10. Procédé selon la revendication 8 ou 9, comprenant de plus l'immersion du corps dans l'eau après l'immersion dans une solution aqueuse alcaline avant le chauffage.

11. Procédé de fabrication d'un os artificiel ostéoinducteur (1),
**caractérisé par**
- une anodisation dans une solution électrolytique d'un corps poreux constitué d'un gros morceau de titane ou d'alliage de titane avec une porosité de 30 à 80 % qui présente
-- des pores (3) communiquant dans un réseau de grille tridimensionnel avec des diamètres de 100 à 3 000 µm et
-- des trous (4) avec un diamètre de 50 µm ou inférieur sur une surface interne des pores (3).
